Europäisches Patentamt

European Patent Office

Office européen des brevets

(11) Publication number: **0 196 687**
**B1**

(12) **EUROPEAN PATENT SPECIFICATION**

(45) Date of publication of patent specification: **10.01.90**

(51) Int. Cl.[5]: **C 07 C 43/20,** C 07 C 41/14

(21) Application number: **86200261.5**

(22) Date of filing: **20.02.86**

(54) Process for the preparation of alkylarylethers.

(30) Priority: **01.03.85 IT 1973485**

(43) Date of publication of application:
**08.10.86 Bulletin 86/41**

(45) Publication of the grant of the patent:
**10.01.90 Bulletin 90/02**

(84) Designated Contracting States:
**AT BE CH DE FR GB LI LU NL SE**

(56) References cited:
**EP-A-0 013 924**
**DE-A-3 339 266**
**DE-C- 827 803**
**FR-A-2 149 461**
**JOURNAL OF THE CHEMICAL SOCIETY, part II,
1947, pages 928-935, The Chemical Society,
London, GB; P.H. GIVEN et al.: "Some catalysed
gas-phase reactions of aromatic hydrocarbons.
Part I. The interaction of benzene with methyl
ether"**

**Ullman's Encyclopaedie der technischen
Chemie, 4 Ed., vol. 8, 1979, pages 334-5**

(73) Proprietor: **ENIRICERCHE S.p.A.**
**Corso Venezia 16**
**I-20121 Milan (IT)**

(72) Inventor: **Brunelli, Maurizio**
**Via A. M. Ampère, 81**
**I-20131 Milan (IT)**
Inventor: **Ferrari, Pierferdinando**
**Via Mazzini, 48**
**I-20090 Segrate (Milan) (IT)**
Inventor: **Stroppa, Fabrizio**
**via Cavalcanti, 1**
**I-20098 San Giuliano Milanese (Milan) (IT)**
Inventor: **Bellussi, Giuseppe**
**Via Valmagini, 19**
**I-29100 Piacenza (IT)**

(74) Representative: **Roggero, Sergio et al**
**Ing. Barzanò & Zanardo Milano S.p.A. Via
Borgonuovo 10**
**I-20121 Milano (IT)**

EP 0 196 687 B1

Courier Press, Leamington Spa, England.

## Description

The invention relates to a process for preparing anisole.

EP—A—0 013 924 discloses a process for preparing arylalkylethers starting from phenols and aliphatic alcohol ethers using a strongly acidic ion-exchange resin: this approach has the defect that it is not possible to work at a high temperature for a long time because the ion-exchange resin would soon lose its physico-chemical and mechanical properties, that which requires a frequent replacement of the resin.

DE—C—827 803 discloses the preparation of mixed methyl ethers from dioxybenzenes and methanol or dimethyl ether the reaction being carried out in the presence of boron phosphate or diatomaceous-earth gel strings soaked in orthophosphoric acid: the use of an inert gas, such as nitrogen, is recommended to prevent overheating and the liquid reactants are dripped, while a nitrogen stream is cocurrently blown through in an open tube. When dimethyl ether is used instead of methanol, pyrocatechol is vaporised in a dimethyl ether stream In a separate vaporiser of 250° and the gaseous mixture passed over the catalyst. No mention of anisole is made in the specification. J. Chem. Society (London), 1947, Part II, pages 928—934 discloses the fact that methanol and dimethylether decompose on the surface of a boron phosphate, and this knowledge is used to carry out reactions of alkylation, rather than etherification, in the gaseous phase.

FR—A—2 149 461 causes dioxybenzenes, thus not monohydric phenol, to react with methanol in the presence of boron phosphate, preferably a water-unstable form of boron phosphate.

Ullmanns Encyclopadie der technischen Chemie, 4th Edn., Vol. *18*, (1979), page 335, merely shows that $BPO_4$ is normally crystalline: this reference discloses a method of preparation giving tetrahedrally crystallized boron phosphate, but does not specify the parameters.

In order to offset the defects and the shortcomings of the prior art, the present invention provides a process for preparing anisole by catalytically reacting phenol and dimethylether in the presence of a solvent in a closed pressurized vessel, characterized in that the reaction is carried out at a temperature of from 280°C to 300°C and under a pressure of at least 4053 kPa (40 atm), in the presence of tetragonal $BPO_4$ having the parameters a=43,39(2) nm and c=66,41(3) nm.

Said boron phosphate is used in amounts by weight comprised within the range of from 0,5% to 50% relative to the weight of the reactants.

By using the tetragonal boron phosphate as defined above, it has been ascertained that best results are obtained in the preparation of anisole, both from the point of view of the yields and the selectivity.

The relevant reaction is conveniently carried out using anhydrous toluene, which is preferred among the aromatic hydrocarbon solvents.

The invention will be better understood with reference to the Examples and the single Figure of the accompanying drawings, wherein:

Example 1 is exemplary of the practical preparation of the tetragonal boron phosphate referred to above: the Figure exhibits a characteristic plot, wherein the abscissa is the angle 2 Ⓝ (Cu Kα=15,418 nm) and the ordinate is the relevant intensity.

Example 2 is a practical example of the best mode of carrying out the reaction.

The process according to the present invention can be carried out, at the operator's option, either batchwise or as a continuous run: If the batchwise way is selected, the boron phosphate is removed by filtering it off from the reaction mass prior to withdrawing the as-formed anisole.

Example 1 (preparation of the boron phosphate catalyst)

1000 g of distilled water are heated to 70°C in a Pyrex (Trade Mark) glass beaker, and are then supplemented with 92,7 g of boric acid, with stirring. Upon complete dissolution of the boric acid, 172,8 g of an 85% aqueous solution of orthophosphoric acid are added, with stirring, and stirring is continued to evaporate off some water until an aqueous slurry is obtained.

The solids are dried at a temperature of 100°C, and then fired at 1000°C during two hours. The calcined solids are then washed four times, each time reslurrying the solids in one litre of boiling distilled water, whereupon the solids are collected on a filter. The solids are now dried and subjected to X-ray diffraction analysis of the powder.

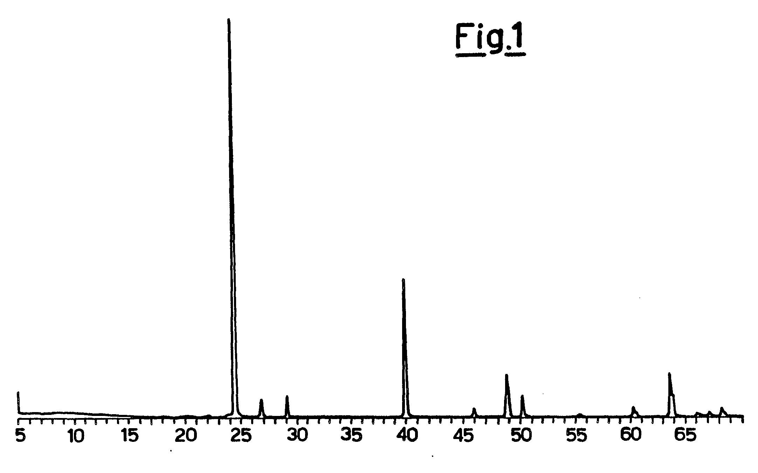

The spectrum shown in Fig. 1 shows that the parameters of the tetragonal boron phosphate so prepared are:

$$a=43,39 \text{ (2) nm}$$
$$c=66,41 \text{ (3) nm.}$$

Example 2

A 200-ml stainless-steel autoclave equipped with a magnetic stirrer, is charged with 22,04 parts by weight of $BPO_4$ prepared according to Example 1, and 50,55 parts of a mixture composed of phenol (24% by weight), dimethylether (DME) (18,99% by weight), toluene (57,01% by weight) and water (0,06% by weight relative to the sum of the weight of all the reactants, solvents and reaction media which make up the reaction mixture.

The reaction is carried out at a temperature of 280°C, under a pressure of 4053 kPa for 48 hours.

The gaschromatographic analysis of the products gave the following results, all on a weight basis:

DME: 14,80%;
Methanol: 1,17%;
Toluene: 57,01%;
Anisole: 14,69%;
Methylanisole: 0,45%;
Phenol: 10,62%;
Cresol: 0,20%, and
Water: 1,06%.

The anisole yield is 53% on a molar basis, and the selectivity to anisole is as high as 96% on a molar basis.

## Claims

1. Process for preparing anisole by catalytically reacting phenol and dimethylether in the presence of a solvent in a closed pressurized reaction vessel, characterized in that the reaction is carried out at a temperature of from 280°C to 300°C and under a pressure of at least 4053 kPa (40 atm), in the presence of tetragonal $BPO_4$ having the parameters a=43,39(2) nm, and c=66,41(3) nm.

2. Process according to claim 1, wherein the solvent is toluene.

## Patentansprüche

1. Verfahren zur Herstellung von Anisol durch katalytische Umsetzung von Phenol und Dimethylether in Anwesenheit eines Lösungsmittels in einem geschlossenen Druckreaktor, dadurch gekennzeichnet, daß die Umsetzung bei einer Temperatur von 280°C bis 300°C und unter einem Druck von wenigstens 4053 kPa (40 atm) in Anwesenheit von tetragonalem $BPO_4$, das die Parameter a=43,39(2) nm und c=66,41(3) nm aufweist, ausgeführt wird.

2. Verfahren nach Anspruch 1, worin das Lösungsmittel Toluol ist.

## Revendications

1. Procédé pour préparer de l'anisole par réaction catalytique de phénol et de diméthyléther en présence d'un solvant dans un réacteur fermé sous pression, caractérisé en ce que la réaction est mise en oeuvre à une température de 280 à 300°C et sous une pression d'au moins 4053 kPa (40 atm), en présence de $BPO_4$ quadratique dont les paramètres sont les suivants: a=43,39(2) nm, et c=66,41(3) nm.

2. Procédé selon la revendication 1, dans lequel le solvant est le toluène.

Fig.1